# EUROPEAN PATENT SPECIFICATION

(11) **EP 1 658 006 B1**
(45) Date of publication and mention of the grant of the patent: **12.05.2010**
(21) Application number: 04764412.5
(22) Date of filing: 24.08.2004
(51) Int. Cl.: A61B 5/0285, A61B 5/022, A61B 5/024

(54) **DEVICE FOR DETECTING ARTERIAL PRESSURE**
VORRICHTUNG ZUM NACHWEIS DES ARTERIENDRUCKS
DISPOSITIF POUR DETECTER LA PRESSION ARTERIELLE

(30) Priority: 29.08.2003 IT MI20031683
(43) Date of publication of application: 24.05.2006
(73) Proprietor: Ghigini, Francesca, 20121 Milano (IT)
(72) Inventor: Ghigini, Francesca, 20121 Milano (IT)
(74) Representative: Modiano, Micaela Nadia
(86) International application number: PCT/EP2004/009433
(87) International publication number: WO 2005/020810

(56) References cited:
- EP-A- 0 472 464
- EP-A- 0 506 520

## Description

### Technical Field

The present invention relates to a device for detecting arterial pressure. More particularly, the invention relates to a device suitable to detect arterial pressure, generally known as sphygmomanometer, which is capable of giving maximum objectivity to the measurement made.

### Background Art

As is known, the sphygmomanometer was introduced at the end of the 19^{th} century and is still universally used today according to the measurement technique perfected in the early 20^{th} century.

Such instrument, composed of a manometer connected to a chamber that can be inflated by means of an air-bulb, is designed to oppose a known pressure to the arterial pressure and therefore allow to read the pressure values when blood flow is detected, by listening with a stethoscope during decompression of the cuff that contrasts the known pressure, and subsequently, by auscultation, the level at which the action of the cuff no longer affects the detected sounds.

The inflatable chamber, integrated in a cuff that is applied to the arm of the patient, produces on the arm a pneumatic pressure that exceeds the arterial pressure at a certain point of the compression, interrupting the flow of blood downstream of the cuff. Once the arterial pressure has been exceeded by 20-30 mm Hg, decompression of the cuff is performed by means of a pneumatic valve that is normally integrated in the bulb.

During decompression, the operator listens to the sounds produced by the artery, detected by means of a stethoscope that is appropriately placed on the arm. In this manner, the operator detects a series of sounds having mutually different tones, intensities and durations, produced by the arterial pulses, which in turn are a consequence of cardiac activity and of the resistance of the arterial vessel. At this point, the operator must determine which of these pulses represents the systolic value (maximum pressure) and which one represents the diastolic value (minimum pressure).

The above described measurement of arterial pressure however suffers drawbacks that are due to the fact that the measurement inherently has the following subjective aspects.

Excessively rapid decompression rate: the error due to an excessively rapid decompression rate in the cuff derives from the reaction time of the operator.

When the operator detects the pulse that determines the systolic value or the one that determines the diastolic value, he/she reads on the graduated scale of the manometer the value, which in the meantime has decreased as a function of its decompression rate.

As a consequence of this, medical doctrine prescribes a rate between 2 and 3 mm Hg per second as regards decompression. However, in practice this prescription is commonly ignored, because there is a tendency to reduce the time required for the measurement, underestimating the error made and therefore failing to obtain reliable data.

A second type of error is given by a parallax error, i.e., by an incorrect placement of the operator with respect to the manometer. As is evident, such error can be opposite in sign and variable in meaning, with random characteristics, indeed depending on the position that the operator assumes with respect to the manometer.

Another error is given by the operator's natural tendency to round off the read value to a value that is easy to memorize. This rounding off is usually performed to 5 mm Hg and sometimes to 10 mm Hg.

Yet another error is given by the uncertainty in determining which pulse indicates systolic pressure and likewise which pulse indicates diastolic pressure.

In the first case, the phenomenon that causes so-called supermaximal pressure is known in the doctrine. When decompression begins, one waits to detect the first pulse in the stethoscope. The first pulse normally indicates the systolic value (maximum pressure). However, the first pulse does not always correspond to flow of blood beneath the inflatable chamber caused by the blood pressure exceeding the pressure that contrasts it by means of the cuff. Actually, with a high-intensity wrist pulse it is possible to detect pulses produced by the impact of the arterial pulsation against the edge of the cuff that constitutes the obstacle that cannot be passed until its pressure has dropped below the level of the arterial pressure.

Substantially, this noise may be audible with a variable acoustic level depending on the intensity of the wrist pulse. In the presence of a "strong" pulsation, these supermaximal pulses must be discriminated by the operator by comparing their intensity with the subsequent pulses.

In the second case, there is uncertainty in determining which pulse can be defined as a diastolic pulse, i.e., the pulse that indicates the minimum pressure. Determination of diastolic pressure has always been more difficult to define, and uniformity of its results is currently unsolved.

Electronic sphygmomanometers are currently known which use the principle of the manual sphygmomanometer but replace auscultation by the operator with detection of the signal by means of a microphone or by detecting the sphygmic pulse in the form of a pneumatic oscillation within the circuit of the instrument, caused by the arterial pulsation itself.

The first method is the closest possible analogy to stethoscopic auscultation, with the difference of replacing human abilities in hearing and processing the signal with electronic means that can offer different results due to obvious inherent characteristics.

The second technique has greater operating differences, since it is not based on the same element described above, i.e., the noise generated by blood flow, but on a different element, such as the pneumatic oscillation inside the circuit of the instrument, caused by the pulsation itself. By processing with increasingly sophisticated algorithms, an attempt has been made to attain a result that is as close as possible to the result that can be obtained with human auscultation, but currently it is not possible to have a completely reliable measurement.

EP-A-0 472 464, accordingly to the respective preambles of claims 1 and 7, discloses a pressure detecting apparatus for sphygmic diagnosis in oriental medicine comprising three pressure sensors for converting the arterial pulses on the three spots into electrical waves and a cuff band which is mounted on the wrist of a patient for biasing the pressure sensors.

### SUMMARY OF THE INVENTION

The aim of the present invention is to provide a device for detecting arterial pressure that substantially allows to improve the precision of the measurement.

Within this aim, an object of the present invention is to provide a device for detecting arterial pressure that allows to eliminate the drawbacks due to an excessively high decompression rate, to parallax error, to the operator's tendency to round off the measured value, and finally to the uncertainty in determining the pulse to be defined as systolic and the pulse to be defined as diastolic.

A further object of the present invention is to provide a device for detecting arterial pressure that maintains the central role of human assessment in pressure measurement, supporting it with the aid of electronic means.

A further object of the present invention is to provide a device for detecting arterial pressure that is highly reliable, relatively simple to manufacture, and at competitive costs.

This aim and these and other objects that will become better apparent hereinafter are achieved by a device for detecting arterial pressure with high measurement precision, comprising a cuff with inflatable chamber, adapted to be placed around the arm of a patient; means for introducing air to inflate said cuff, and decompression means suitable to decompress said inflatable chamber, **characterized in that** it comprises means adapted to detect and store all the sphygmic pulses generated by arterial pressure and to identify the pulses that correspond to the appearance and disappearance of the pulse, detected by means of a technique for detecting sphygmic pulses generated by arterial pressure that requires the intervention and judgment of an operator.

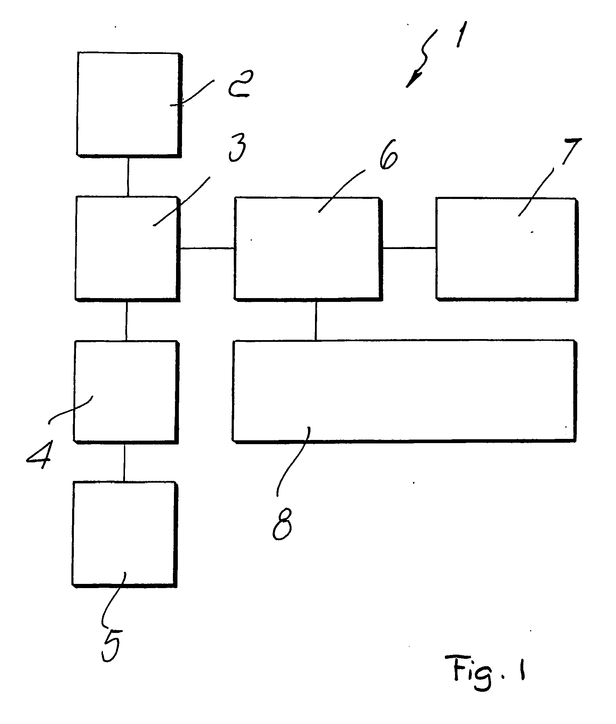

### Brief Description of the Drawing

Further characteristics and advantages of the invention will become better apparent from the description of a preferred but not exclusive embodiment of the device according to the present invention, illustrated by way of non-limiting example in the accompanying drawing, wherein the only figure is a block diagram of the apparatus according to the present invention.

### Ways of Carrying out the Invention

With reference to the figure, the device according to the present invention, generally designated by the reference numeral 1, comprises air pumping means 2, for example of the manual or electric type, which are connected to a cuff 3, inside which an inflatable chamber is accommodated. Decompression means 4, conveniently constituted by a compression valve, are adapted to perform a constant and controlled decompression of the cuff 3, as prescribed by medical doctrine to perform an accurate measurement.

The device further comprises means 5 for complete and instantaneous venting of the air of the cuff 3. The venting means 5 are constituted, for example, by a valve that allows to discharge instantly, at the operator's choice, the air from the cuff 3. As an alternative, it is possible to use as a vent the same decompression valve, used appropriately.

The device according to the invention further has pressure transducer means 6, which are adapted to detect electronically all the sphygmic pulses generated by arterial pulsation and are connected to storage means 7 adapted to store said pulses. The means 6 for detecting the sphygmic pulses further allow to identify the pulses that correspond to the appearance and disappearance of the pulse, detected by means of any detection method that provides for the intervention of the operator and for his subjective judgment.

The transducer means continuously detect the value of the pressure of the cuff, like a normal manometer does. This value is reported in real time by the display and recorded together with the detection of the sphygmic pulses. Accordingly, once the measurement has ended, the operator can analyze, on the display, a listing in which, next to the pressure values expressed for example in mm Hg (or KPa), he/she can read, at the pressure pulses, a value that indicates their sphygmic intensity.

The operator, when he perceives the stethoscope pulses (or pulses detected in another manner) that correspond to the systolic and diastolic pressure, presses a button of the device to "mark" these values on the digital scale of the device.

Substantially, therefore, the user performs a manual detection of the pulses by means of the conventional stethoscope (or, as mentioned, by means of any other method), but the means 6 for detecting sphygmic pulses are associated with said detection and allow to identify the pulses that correspond to systolic pressure and to diastolic pressure.

Storage of the data, i.e., of the pulses and therefore of the chart, allows to determine with certainty the pulses that actually correspond to the maximum and minimum values of arterial pressure, performing this analysis after detection by means of the stethoscope.

Substantially, therefore, the operator has control over the measurements performed manually, and accordingly can associate the precision of manual detection with a device that allows to eliminate the previously mentioned uncertainties that are inherent in the measurement.

The device is further provided with display means 8, which are adapted to display at least the following detected pressure levels: in real time, at the stored times, with previous measurements (i.e., measurement history).

The device according to the present invention is capable of transferring the detected results to other storage and/or printout means.

It should be noted that the measurement method currently in use, i.e., with a cuff provided with an inflatable chamber, prescribes the use of an inflatable chamber that is proportionate to the size of the arm of the patient. However, since in practice it is not possible to have a cuff for each patient, medical doctrine has established three types of cuffs for three different types of patient. However, very often an operator does not comply with these indicators and always uses a single standard cuff.

The use of a standard cuff, however, is another source of error, which adds to the previously described errors that are inherent in the measurement.

For this purpose, the device according to the present invention uses a cuff 3 on which a scale is printed along its entire longitudinal side, said scale indicating, when the cuff is applied, the circumference of the arm of the user. This information can be used, by entering it in the device according to the invention, as a corrective factor for the arterial pressure measurement that is made, accordingly complying with the criteria of using a cuff for each type of patient.

In practice it has been found that the device according to the present invention allows to perform a manual measurement with a stethoscope of the arterial pressure of a patient, with the association of means adapted to detect the sphygmic pulses generated by arterial pressure and to then identify the pulses that correspond to the appearance and disappearance of the pulse detected by means of the stethoscopic technique. These pulses correspond respectively to systolic pressure and to diastolic pressure.

The operator, therefore, can perform a manual measurement with the aid of a sort of electronic measurement for control and confirmation. The device according to the invention allows to store the chart of the pulses in order to perform a subsequent verification thereof, so as to determine assuredly the pulses that actually correspond to the maximum and minimum values of the arterial pressure.

The device thus conceived is susceptible of numerous modifications and variations, within the scope of the appended claims. All the details may be replaced with other technically equivalent elements.

In practice, the materials used, as well as the contingent shapes and dimensions, may be any according to requirements and to the state of the art.

## Claims

1. A device (1) for detecting arterial pressure with high measurement precision, comprising a cuff (3) with inflatable chamber, adapted to be placed around the arm of a patient, means (2) for introducing air to inflate said cuff (3), decompression means (4) adapted to decompress said inflatable chamber, and means (6) adapted to electronically detect and store all the sphygmic pulses generated by the arterial pulsation and to identify the pulses that correspond to the appearance and disappearance of the pulse, **characterized in that** it further comprises a button that is adapted to be pressed by an operator when the operator manually detects sphygmic pulses that correspond to the systolic or diastolic pressure using a conventional stethoscope, the pressing of said button of the device (1) marking said manually detected sphygmic on said stored pulses for a later intervention and subjective judgment of an operator.

2. The device (1) according to claim 1, **characterized in that** said cuff (3) comprises a scale which is printed along its entire longitudinal side, said scale indicating, when the cuff (3) is applied, the circumference of the arm of the user which is used by the device (1) as a corrective factor for the arterial pressure measurement that is made, accordingly complying with the criteria of using a cuff for each type of patient.

3. The device (1) according to claim 1, **characterized in that** said decompression means of said inflatable chamber comprise a valve for providing constant and time-controlled decompression.

4. The device (1) according to claim 1, **characterized in that** it comprises discharge means adapted to completely and instantaneously discharge the inflatable chamber of said cuff (3).

5. The device (1) according to one or more of the preceding claims, **characterized in that** said means for detecting and storing the sphygmic pulses are connected to data storage means, which are adapted to store the chart of the sphygmic pulses.

6. The device (1) according to one or more of the preceding claims, **characterized in that** it comprises a display that is adapted to display the detected levels of pressure and the levels of sphygmic intensity of the pulsations.

7. A method for detecting arterial pressure, comprising the steps of:
pumping air into a cuff (3) provided with an inflatable chamber;
decompressing said inflatable chamber;
electronically detecting and storing all the sphygmic pulses generated by arterial pulsation by using an electronic sensing and storage circuit;
manually detecting, using a conventional stethoscope with the intervention and subjective judgment of an operator, the sphygmic pulses that correspond respectively to the appearance and disappearance of the pulse,
**characterized in that** it comprises the steps of:
marking by pressing a button of the device (1) said electronically detected and stored sphygmic pulses when the operator manually detects said sphygmic pulses using said conventional stethoscope
identifying, among said sphygmic pulses electronically detected and stored, the ones that correspond to the appearance and disappearance of the pulse beat, detected by means of said stethoscope.

8. The method according to claim 7, **characterized in that** said step of performing the decompression of said inflatable chamber comprises the execution of decompression at a controlled and constant rate.

9. The method according to one or more of claims 7 and 8, **characterized in that** it comprises a step of storing said sphygmic pulses generated by arterial pulsation, in order to allow subsequent analysis of the chart of sphygmic pulses, in order to determine assuredly the pulses that actually correspond to the maximum and minimum values of arterial pressure.

10. The method according to one or more of claims 7 to 9, **characterized in that** it comprises a step of measuring the circumference of the arm of the user with a scale which is printed along the entire longitudinal side of said cuff (3).

11. The method according to claim 10, **characterized in that** it further comprises the step of entering in the device (1) the circumference of the arm of the user as a corrective factor for the arterial pressure measurement that is made, so as to comply with the criteria of using a cuff (3) for each type of patient.

## Patentansprüche

1. Vorrichtung (1) für das Ermitteln des arteriellen Drucks mit hoher Messgenauigkeit, wobei die Vorrichtung eine Stulpe (3) mit aufblasbarer Kammer, die dazu geeignet ist, um um den Arm eines Patienten platziert zu werden, eine Belüftungseinrichtung (2) zum Aufpumpen der Stulpe (3), eine Druckreduzierungseinrichtung (4), die geeignet ist, die aufblasbare Kammer zu dekomprimieren, sowie eine Einrichtung (6), die geeignet ist, alle vom arteriellen Pulsschlag erzeugten Sphygmo-Pulse elektronisch zu ermitteln und zu speichern sowie die Pulse aufzuzeigen, die dem Auftreten und Ausbleiben des Pulsschlages entsprechen, aufweist, **dadurch gekennzeichnet, dass** die Vorrichtung ferner einen Knopf aufweist, der geeignet ist, um von einem Bediener gedrückt zu werden, sobald der Bediener manuell unter Verwendung eines handelsüblichen Stethoskops Shpygmo-Pulse, die einem systolischen oder diastolischen Druck entsprechen, ermittelt, wobei das Drücken des Knopfes an der Vorrichtung (1) ein Markieren der von Hand ermittelten Sphygmo-Pulse an den gespeicherten Pulsen für einen späteren Eingriff und eine subjektive Beurteilung durch einen Bediener bewirkt.

2. Vorrichtung (1) nach Anspruch 1, **dadurch gekennzeichnet, dass** die Stulpe (3) eine Messskala, die entlang der gesamten Längsseite gezeichnet ist, aufweist, und die Messskala, sobald die Stulpe (3) angelegt ist, den Armumfang des Patienten anzeigt, der von der Vorrichtung (1) als ein Korrekturfaktor für die Messung des arteriellen Drucks verwendet wird, und die Anwendungskriterien zum Verwenden einer Stulpe für jeden Typ von Patienten entsprechend erfüllt.

3. Vorrichtung (1) nach Anspruch 1, **dadurch gekennzeichnet, dass** die Druckreduzierungseinrichtung der aufblasbaren Kammer ein Ventil zur Ausführung einer konstanten und zeitkontrollierten Dekompression aufweist.

4. Vorrichtung (1) nach Anspruch 1, **dadurch gekennzeichnet, dass** die Vorrichtung eine Entlüftungseinrichtung aufweist, die geeignet ist, die aufblasbare Kammer der Stulpe (3) vollständig und unverzögert zu entlüften.

5. Vorrichtung (1) nach wenigstens einem der vorhergehenden Ansprüche, **dadurch gekennzeichnet, dass** die Einrichtung zum Ermitteln und Speichern der Sphygmo-Pulse mit einer Datenspeichereinrichtung, die dazu geeignet ist, eine Aufstellung der Sphygmo-Pulse zu speichern, verbunden ist.

6. Vorrichtung (1) nach wenigstens einem der vorhergehenden Ansprüche, **dadurch gekennzeichnet, dass** die Vorrichtung eine Anzeige aufweist, die geeignet ist, die ermittelten Druckstufen und Stufen der Sphygmo-Intensität der Pulsschläge anzuzeigen.

7. Ein Verfahren zum Ermitteln des arteriellen Drucks, das die folgenden Schritte aufweist:
das Pumpen von Luft in eine Stulpe (3), die eine aufblasbare Kammer aufweist;
das Dekomprimieren der aufblasbaren Kammer;
das elektronische Ermitteln und Speichern aller vom arteriellen Pulsschlag erzeugten Sphygmo-Pulse mittels Verwendung einer elektronischen Abtast- und Speichereinheit;
das manuelle Ermitteln von Sphygmo-Pulsen unter Verwendung eines handelsüblichen Stethoskops mittels Eingriff und subjektiver Beurteilung eines Bedieners, die dem Auftreten und Ausbleiben des Pulsschlages entsprechen,
**dadurch gekennzeichnet, dass** das Verfahren die folgenden Schritte umfasst:
das Markieren mittels Drücken eines Knopfes an der Vorrichtung (1) der elektronisch ermittelt und gespeicherten Sphygmo-Pulse, sobald der Bediener manuell unter Verwendung eines handelsüblichen Stethoskops Sphygmo-Pulse ermittelt,
das Aufzeigen nicht nur der elektronisch ermittelt und gespeicherten Sphygmo-Pulse, sondern auch derjenigen Pulse, die dem Auftreten und Ausbleiben des mit Hilfe eines Stethoskops ermittelten Pulsschlages korrespondieren.

8. Verfahren nach Anspruch 7, **dadurch gekennzeichnet, dass** der Verfahrensschritt, der die Dekompression der aufblasbaren Kammer betrifft, eine Ausführung der Dekompression zu einer kontrollierten und konstanten Rate aufweist.

9. Verfahren nach wenigstens einem der vorhergehenden Ansprüche 7 und 8, **dadurch gekennzeichnet, dass** das Verfahren einen Verfahrensschritt zur Speicherung der vom arteriellen Pulsschlag erzeugten Sphygmo-Pulse aufweist, der es ermöglicht, die Aufstellung der Sphygmo-Pulse zu einem späteren Zeitpunkt zu analysieren und die Pulse, die tatsächlich den höchsten und niedrigsten Werten des arteriellen Drucks entsprechen, sicher zu bestimmen.

10. Verfahren nach wenigstens einem der vorhergehenden Ansprüche 7 bis 9, **dadurch gekennzeichnet, dass** das Verfahren einen Verfahrensschritt zur Messung des Armumfangs eines Patienten mittels einer Messskala, die entlang der gesamten Längsseite der Stulpe (3) gezeichnet ist, aufweist.

11. Verfahren nach Anspruche 10, **dadurch gekennzeichnet, dass** das Verfahren ferner einen Verfahrensschritt zur Eingabe des Armumfangs eines Patienten in die Vorrichtung (1) als Korrekturfaktor für die durchzuführende Messung des arteriellen Drucks aufweist, um die Anwendungskriterien zum Verwenden einer Stulpe (3) für jeden Typ von Patient zu erfüllen.

## Revendications

1. Dispositif (1) pour détecter la pression artérielle avec une grande précision de mesure, comprenant un brassard (3) avec une chambre gonflable, adapté pour être placé autour du bras d'un patient, des moyens (2) pour introduire de l'air afin de gonfler ledit brassard (3), des moyens de décompression (4) adaptés pour décomprimer ladite chambre gonflable et des moyens (6) adaptés pour détecter et stocker par voie électronique toutes les pulsations sphygmiques générées par la pulsation artérielle et pour identifier les pulsations qui correspondent à l'apparition et à la disparition du pouls, **caractérisé en ce qu'**il comprend en outre un bouton qui est adapté pour être pressé par un opérateur lorsque l'opérateur détecte manuellement des pulsations sphygmiques qui correspondent à la pression systolique ou diastolique en utilisant un stéthoscope usuel, la pression dudit bouton du dispositif (1) marquant ladite pulsation sphygmique manuellement détectée sur lesdites pulsations stockées pour une intervention ultérieure et le jugement subjectif d'un opérateur.

2. Dispositif (1) selon la revendication 1, **caractérisé en ce que** ledit brassard (3) comprend une échelle qui est imprimée sur toute sa longueur, ladite échelle indiquant, lorsque le brassard (3) est appliqué, la circonférence du bras de l'utilisateur qui est utilisée par le dispositif (1) comme facteur de correction pour la mesure de la pression artérielle qui est effectuée, en se conformant par suite aux critères d'utilisation d'un brassard pour chaque type de patient.

3. Dispositif (1) selon la revendication 1, **caractérisé en ce que** lesdits moyens de décompression de ladite chambre gonflable comprennent une soupape pour fournir une décompression constante et contrôlée dans le temps.

4. Dispositif (1) selon la revendication 1, **caractérisé en ce qu'**il comprend des moyens de décharge adaptés pour décharger de manière complète et instantanée la chambre gonflable dudit brassard (3).

5. Dispositif (1) selon une ou plusieurs des revendications précédentes, **caractérisé en ce que** lesdits moyens pour détecter et stocker les pulsations sphygmiques sont reliés à des moyens de stockage de données qui sont adaptés pour stocker le diagramme des pulsations sphygmiques.

6. Dispositif (1) selon une ou plusieurs des revendications précédentes, **caractérisé en ce qu'**il comprend un affichage qui est adapté pour afficher les niveaux détectés de pression et les niveaux d'intensité sphygmique des pulsations.

7. Procédé pour détecter la pression artérielle, comprenant les étapes consistant à :
pomper de l'air dans un brassard (3) muni d'une chambre gonflable ; décomprimer ladite chambre gonflable ;
détecter et stocker par voie électronique toutes les pulsations sphygmiques générées par la pulsation artérielle en utilisant un circuit de détection et de stockage électronique ;
détecter manuellement, en utilisant un stéthoscope usuel avec l'intervention et le jugement subjectif d'un opérateur, les pulsations sphygmiques qui correspondent respectivement à l'apparition et à la disparition du pouls,
**caractérisé en ce qu'**il comprend les étapes consistant à :
marquer par pression d'un bouton du dispositif (1) lesdites pulsations sphygmiques détectées et stockées par voie électronique lorsque l'opérateur détecte manuellement lesdites pulsations sphygmiques en utilisant ledit stéthoscope usuel,
identifier, parmi lesdites pulsations sphygmiques détectées et stockées par voie électronique, celles qui correspondent à l'apparition et à la disparition du battement du pouls, détecté à l'aide dudit stéthoscope.

8. Procédé selon la revendication 7, **caractérisé en ce que** ladite étape consistant à effectuer la décompression de ladite chambre gonflable comprend l'exécution de la décompression à une vitesse contrôlée et constante.

9. Procédé selon une ou plusieurs des revendications 7 et 8, **caractérisé en ce qu'**il comprend une étape de stockage desdites pulsations sphygmiques générées par la pulsation artérielle, afin de permettre une analyse suivante du diagramme des pulsations sphygmiques, afin de déterminer avec certitude les pulsations qui correspondent réellement aux valeurs maximale et minimale de la pression artérielle.

10. Procédé selon une ou plusieurs des revendications 7 à 9, **caractérisé en ce qu'**il comprend une étape de mesure de la circonférence du bras de l'utilisateur avec une échelle qui est imprimée sur toute la longueur dudit brassard (3).

11. Procédé selon la revendication 10, **caractérisé en ce qu'**il comprend en outre l'étape consistant à faire entrer dans le dispositif (1) la circonférence du bras de l'utilisateur comme facteur de correction pour la mesure de la pression artérielle qui est faite en sorte de se conformer aux critères d'utilisation d'un brassard (3) pour chaque type de patient.
